# EUROPEAN PATENT APPLICATION

(11) **EP 4 317 081 A1**
(43) Date of publication of application: **07.02.2024**
(21) Application number: 22779350.2
(22) Date of filing: 11.01.2022
(51) Int. Cl.: C02F 1/00, G06Q 10/00

(54) **CONTROL DEVICE, WATER QUALITY MANAGEMENT SYSTEM, WATER QUALITY MANAGEMENT UNIT, AND WATER QUALITY SENSOR UNIT**

(30) Priority: 31.03.2021 JP 2021060487
(71) Applicant: Niterra Co., Ltd., Nagoya-shi, Aichi 461-0005 (JP)
(72) Inventor: KAMEI Shunsuke, Nagoya-shi, Aichi 467-8525 (JP); OSAWA Norimasa, Nagoya-shi, Aichi 467-8525 (JP); YAZAWA Katsunori, Nagoya-shi, Aichi 467-8525 (JP)
(74) Representative: Grünecker Patent- und Rechtsanwälte PartG mbB
(86) International application number: PCT/JP2022/000521
(87) International publication number: WO 2022/209117

(57) **Abstract**

A control apparatus (10) can be connected to a water quality sensor (30). The control apparatus (10) includes a plurality of input ports (20). The water quality sensor (30) that can be disposed in a plurality of tanks (90) whose number is equal to or less than the number of the input ports (20) is detachably connected the input ports (20). Each of the input ports (20) is related to any one of the plurality of tanks (90). The control apparatus (10) receives a measurement value from the water quality sensor (30) connected to one of the input ports (20), and stores or outputs to the outside the measurement value and a piece of identification information of the input port (20) through which the measurement value is received, in such a manner that the measurement value and the piece of identification information of the input port are related to each other.

## Description

### TECHNICAL FIELD

The present disclosure relates to a control apparatus, a water quality management system, a water quality management unit, and a water quality sensor unit.

### BACKGROUND ART

Patent Literature 1 discloses a product management system for managing growth of an aquatic organism. This product management system includes a control apparatus and a sensor. The sensor measures a piece of information relating to the quality of breeding water in a tank. A measurement value output from the sensor is input to the control apparatus.

### CITATION LIST

### PATENT LITERATURE

Patent Literature 1: JP2021-13361A

### SUMMARY OF INVENTION

### TECHNICAL PROBLEM

In a system of this type, managing pieces of information relating to water quality and obtained at a plurality of tanks is desired.

However, if an individual management apparatus is provided for each of the plurality of tanks, cost is high, and an operator must provide a large amount of labor for each management apparatus.

In view of the above, an object of the present disclosure is to provide a technique for enabling management of pieces of information relating to water quality and obtained at a plurality of tanks, while suppressing an increase in cost and reducing the labor of an operator.

### SOLUTION TO PROBLEM

(1) A control apparatus of the present invention can be connected to a water quality sensor. The control apparatus of the present invention has a plurality of input ports. The water quality sensor that can be disposed in a plurality of tanks whose number is equal to or less than the number of the input ports is detachably connected to the above-described input ports. Each of the above-described input ports is related to any one of the above-described plurality of tanks. The control apparatus of the present invention receives a measurement value from the above-described water quality sensor connected to one of the input ports, and stores or outputs to the outside the above-described measurement value and a piece of identification information of the above-described input port through which the above-described measurement value is received, in such a manner that the measurement value and the piece of identification information of the input port are related to each other.

This control apparatus can store or output to the outside the measurement value from the water quality sensor in such a manner that the measurement value and the piece of identification information of the input port through which the measurement value is received are related to each other. Therefore, pieces of water quality information based on measurement values obtained at the plurality of tanks can be managed individually by a single control apparatus on a tank-by-tank basis. Accordingly, by virtue of this configuration, it is possible to manage pieces of information relating to water quality and obtained at a plurality of tanks, while suppressing an increase in cost and reducing the labor of an operator.

(2) The above-described plurality of input ports of the above-described control apparatus may be identical in shape.

This control apparatus enables connection of the same water quality sensor to the plurality of input ports as a common water quality sensor. Therefore, the number of water quality sensors needed for measurement can be reduced.

(3) The above-described control apparatus may be configured such that it can receive a piece of type information from the above-described water quality sensor connected to the above-described input port, the piece of type information representing a type of the above-described water quality sensor.

By virtue of this configuration, it is possible to manage pieces of water quality information in consideration of the type of the water quality sensor.

(4) A water quality management system of the present invention comprises the above-described control apparatus, and a storage terminal which stores pieces of water quality information based on the measurement values output from the control apparatus, in such a manner that the pieces of water quality information are distinguished from each other on the basis of the pieces of identification information of the input ports through which the measurement values are received.

By virtue of this water quality management system, the pieces of water quality information based on the measurement values output from the control apparatus can be stored in such a manner that the pieces of water quality information are automatically distinguished from one another on the basis of pieces of identification information of the input ports through which measurement values are received. Therefore, it is possible to easily manage the pieces of water quality information obtained from the tanks individually.

(5) The above-described storage terminal may be configured such that it can set a measurement mode to a permanent mode in which the above-described water quality sensor is permanently connected to one of the above-described input ports, or a spot mode in which the above-described water quality sensor is connected to one of the above-described input ports when necessary.

By virtue of this configuration, it is possible to perform more appropriate control depending on whether the measurement mode is the permanent mode or the spot mode.

(6) The above-described storage terminal may be configured such that, in the case where the storage terminal determines that the water quality sensor is not connected in the permanent mode, the storage terminal reports an anomaly.

By virtue of this configuration, the storage terminal can report an anomaly in the case where the storage terminal determines that the water quality sensor is not connected despite the measurement mode being the permanent mode.

(7) The above-described storage terminal may be configured such that it enables setting for establishing correspondence relationships between pieces of identification information of the above-described tanks and pieces of identification information of the above-described control apparatus and the above-described input ports.

By virtue of this configuration, the storage terminal can manage the pieces of water quality information based on the measurement values received from the control apparatus while relating the pieces of water quality information to the tanks.

(8) A plurality of the above-described control apparatuses may be provided. The above-described storage terminal may have a display section and may be configured to cause the display section to display pieces of water quality information based on the above-described measurement values received from the above-described control apparatuses, respectively.

By virtue of this configuration, the pieces of water quality information based on the measurement values output from the plurality of control apparatuses can be displayed by the display section of the storage terminal. Therefore, a managing person can check, by using the storage terminal, the pieces of water quality information obtained at the respective tanks.

(9) The above-described storage terminal may be configured such that it can set measurement conditions for the above-described control apparatus, under which the above-described water quality sensor connected to the above-described control apparatus performs measurement.

By virtue of this configuration, the conditions under which the water quality sensor performs measurement can be set not on the control apparatus side but on the storage terminal side. Therefore, the managing person can set the measurement conditions at the respective tanks by using the storage terminal, and, thus, it is possible to eliminate time and labor; such as for instructing a person at the site to set the measurement conditions.

(10) The above-described storage terminal may be configured such that, in the case where it determines that the piece of water quality information based on the above-described measurement value from the above-described water quality sensor is anomalous, the storage terminal outputs an anomaly signal.

By virtue of this configuration, it is possible to report an anomaly to an external apparatus in the case where the measurement value from the water quality sensor is an anomalous value.

(11) A water quality management unit of the present invention comprises the above-described water quality sensor and the above-described water quality management system.

(12) A water quality sensor unit of the present invention comprises the above-described water quality sensor and the above-described control apparatus.

### ADVANTAGEOUS EFFECTS OF INVENTION

The present invention enables management of pieces of information relating to water quality and obtained at a plurality of tanks, while suppressing an increase in cost and reducing the labor of an operator.

### BRIEF DESCRIPTION OF DRAWINGS

[FIG. 1] FIG. 1 is a configuration diagram conceptually illustrating a water quality management unit.
[FIG. 2] FIG. 2 is a block diagram schematically illustrating functions of a control apparatus.
[FIG. 3] FIG. 3 is a block diagram schematically illustrating functions of a storage terminal.
[FIG. 4] FIG. 4 is an explanatory chart showing an example of a display image for establishing correspondence relationships between pieces of identification information of tanks and pieces of identification information of control apparatuses and input ports.
[FIG. 5] FIG. 5 is an explanatory chart showing an example of a display image for setting measurement conditions.
[FIG. 6] FIG. 6 is an explanatory chart showing an example of a display image for displaying, for each tank, a state corresponding thereto.
[FIG. 7] FIG. 7 is an explanatory chart showing an example of a display image for displaying water quality information of a selected tank.
[FIG. 8] FIG. 8 is a configuration diagram conceptually illustrating a water quality management system including production-site-side apparatuses.
[FIG. 9] FIG. 9 is a block diagram schematically illustrating functions of each production-site-side apparatus.

### DESCRIPTION OF EMBODIMENTS

### 1. First embodiment

### 1-1. Configuration of water quality management unit

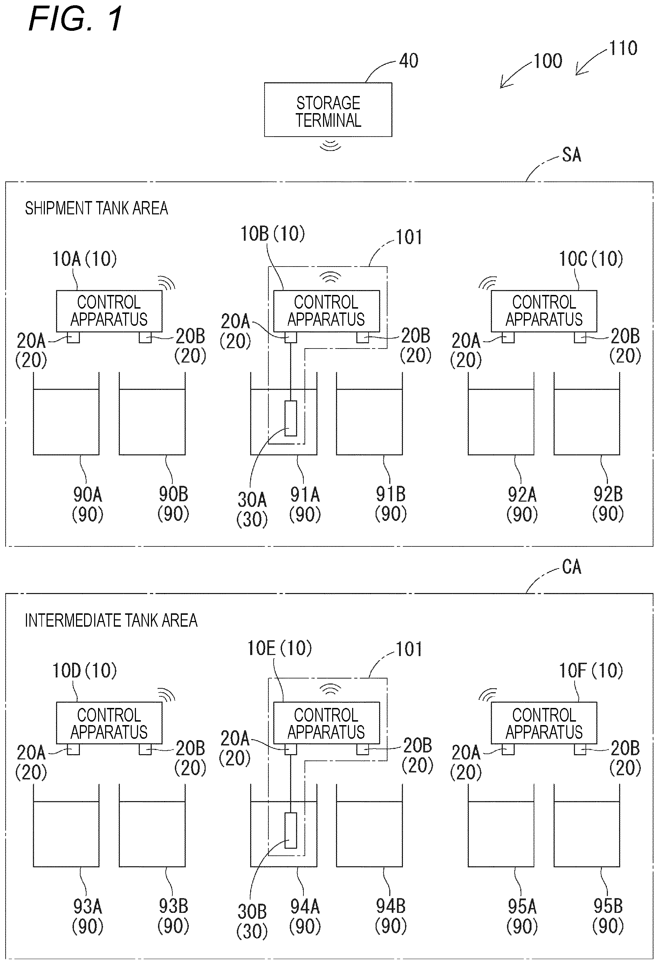

A water quality management unit 110 shown in FIG. 1 includes a water quality management system 100 and a plurality of water quality sensors 30 (water quality sensors 30A and 30B in the example shown in FIG. 1).

The water quality management system 100 is a system for managing the quality of breeding water in tanks 90 in which aquatic organisms are grown. The water quality management system 100 can manage the quality of breeding water in a plurality of tanks 90. In the example shown in FIG. 1, a production site where the tanks 90 are disposed includes a shipment tank area SA and an intermediate tank area CA. Tanks 90A, 90B, 91A, 91B, 92A, and 92B are disposed in the shipment tank area SA. Tanks 93A, 93B, 94A, 94B, 95A, and 95B are disposed in the intermediate tank area CA.

The water quality management system 100 includes a plurality of control apparatuses 10 (control apparatuses 10A, 10B, 10C, 10D, 10E, and 10F in the example shown in FIG. 1) and a storage terminal 40. The control apparatuses 10 constitute water quality sensor units 101 together with the above-described water quality sensors 30.

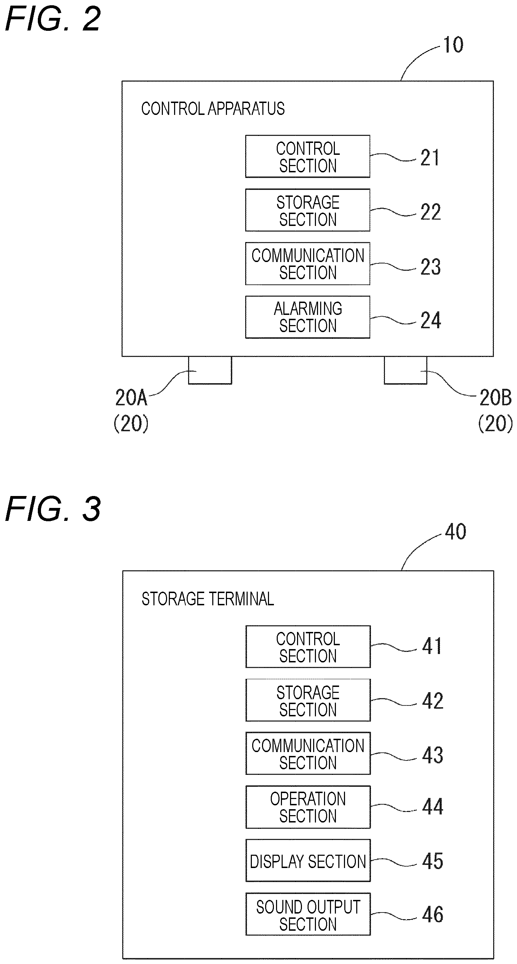

As shown in FIGS. 1 and 2, each control apparatus 10 includes a plurality of input ports 20 (input ports 20A and 20B in the example shown in FIG. 1), a control section 21, a storage section 22, a communication section 23, and an alarming section 24.

The water quality sensors 30 are detachably connected to the input ports 20. The plurality of input ports 20 are identical in shape. Therefore, the control apparatus 10 enables connection of the same water quality sensor 30 to any of the input ports 20.

The control section 21 is, for example, an information processing apparatus including a CPU and can perform various types of computations, controls, and information processing. The storage section 22 is, for example, a memory such as ROM, RAM, or the like and stores various pieces of information. The communication section 23 is an apparatus which communicates with an external apparatus in accordance with a known scheme. Although the communication scheme employed in the present embodiment is radio communication, the communication scheme may be wire communication. The alarming section 24 has a function of reporting an anomaly when it occurs and is, for example, a display lamp, a speaker, or the like. The alarming section 64 reports an anomaly by, for example, lighting the display lamp in a predetermined display color or outputting a sound by the speaker.

Each water quality sensor 30 is, for example, a multiparameter water quality meter which can measure a plurality of items (parameters) regarding water quality. The parameters may be physical parameters such as the temperature of the breeding water, the flow speed of the breeding water, the redox potential of the breeding water, and the conductivity of the breeding water. Alternatively, the parameters may be chemical parameters such as the dissolved oxygen concentration of the breeding water, the pH of the breeding water, the salinity concentration of the breeding water, the calcium concentration of the breeding water, the magnesium concentration of the breeding water, the ammonia concentration of the breeding water, the nitrite concentration of the breeding water, and the nitric acid concentration of the breeding water. When a measurement condition is satisfied, the water quality sensor 30 measures the parameters and outputs measurement values. A piece of type information which represents the type of the water quality sensor 30 is stored in the water quality sensor 30 beforehand. Therefore, the water quality sensor 30 can output the piece of type information. No limitation is imposed on the timing when the water quality sensor 30 outputs the piece of type information. The timing when the water quality sensor 30 outputs the piece of type information may be, for example, the time when the water quality sensor 30 is connected to the control apparatus 10 or the time when the water quality sensor 30 outputs the measurement values.

The storage terminal 40 is configured, for example, as a computer and includes a control section 41, a storage section 42, a communication section 43, an operation section 44, a display section 45, and a sound output section 46. The control section 41 is, for example, an information processing apparatus including a CPU and can perform various types of computations, controls, and information processing. The storage section 42 is, for example, a memory such as ROM, RAM, or the like and stores various pieces of information. The communication section 43 is an apparatus which communicates with an external apparatus in accordance with a known scheme. Although the communication scheme employed in the present embodiment is radio communication, the communication scheme may be wire communication. The operation section 44 includes well known input devices such as a keyboard, a mouse, a touch panel, etc. The display section 45 is a well known display apparatus such as a display. The sound output section 46 is, for example, a speaker.

### 1-2. Preparation before measurement

The input ports 20 of the control apparatuses 10 are prepared in such a manner that one input port is provided for one tank 90. For example, the input port 20A of the control apparatus 10A is provided for the tank 90A, and the input port 20B of the control apparatus 10A is provided for the tank 90B. The water quality sensor 30 connected to a certain input port 20 is disposed in a tank 90 corresponding to the certain input port 20 and measures the quality of the breeding water in the tank 90. For example, the water quality sensor 30 connected to the input port 20A of the control apparatus 10A is disposed in the tank 90A, which corresponds to the input port 20A of the control apparatus 10A, and measures the quality of the breeding water in the tank 90A. Also, the water quality sensor 30 connected to the input port 20B of the control apparatus 10A is disposed in the tank 90B, which corresponds to the input port 20B of the control apparatus 10A, and measures the quality of the breeding water in the tank 90B. Similarly, the input ports 20 of the control apparatuses 10B, 10C, 10D, 10E, and 10F are provided in such a manner that they correspond to the tanks 91A, 91B, 92A, 92B, 93A, 93B, 94A, 94B, 95A, and 95B, respectively.

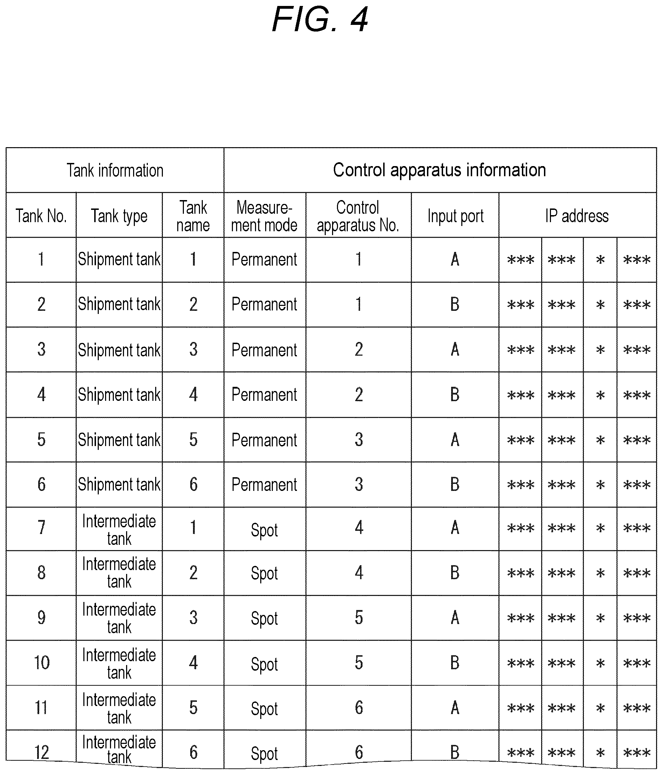

In the storage terminal 40, a setting for establishing a correspondence relationship between a piece of identification information of each tank 90 and pieces of identification information of the control apparatus 10 and the input port 20 provided for that tank 90. FIG. 4 shows an example of a display image for establishing correspondence relationships between pieces of identification information of the tanks 90 and pieces of identification information of the control apparatuses 10 and the input ports 20.

In the example shown in FIG. 4, the piece of identification information of each control apparatus 10 is "control apparatus No." and "IP address." An arbitrary number is entered in each input field for "control apparatus No." The IP address of the control apparatus 10 is entered in each input field for "IP address." In the example shown in FIG. 4, the piece of identification information of each input port 20 is "input port." "A" or "B" is entered in each input field for "input port."

In the example shown in FIG. 4, the piece of identification information of each tank 90 is "tank information"; more specifically, "tank No.," "tank type," and "tank name." Consecutive numbers are entered in input fields for "tank No." An arbitrary character string is entered in each input field for "tank type"; specifically, "shipment tank," "intermediate tank," or the like is entered. An arbitrary character string is entered in each input field for "tank name." In the example shown in FIG. 4, a number is entered in each input field for "tank name."

A managing person can establish the correspondence relationships between the pieces of identification information of the tanks 90 and the pieces of identification information of the control apparatuses 10 and the input ports 20 by inputting various pieces of information by using the operation section 44. As a result, correspondence relationship data obtained by establishing the correspondence relationships between the pieces of identification information of the tanks 90 and the pieces of identification information of the control apparatuses 10 and the input ports 20 are stored in the storage terminal 40.

As shown in FIG. 4, in the storage terminal 40, a measurement mode is set for each input port 20. The measurement mode is "permanent mode" or "spot mode." The permanent mode is a measurement mode in which water quality is measured by the water quality sensor 30 permanently connected to the input port 20. The "spot mode" is a measurement mode in which, when measurement is performed, the water quality sensor 30 is connected to the input port 20 to measure water quality.

### 1-3. Measurement of water quality

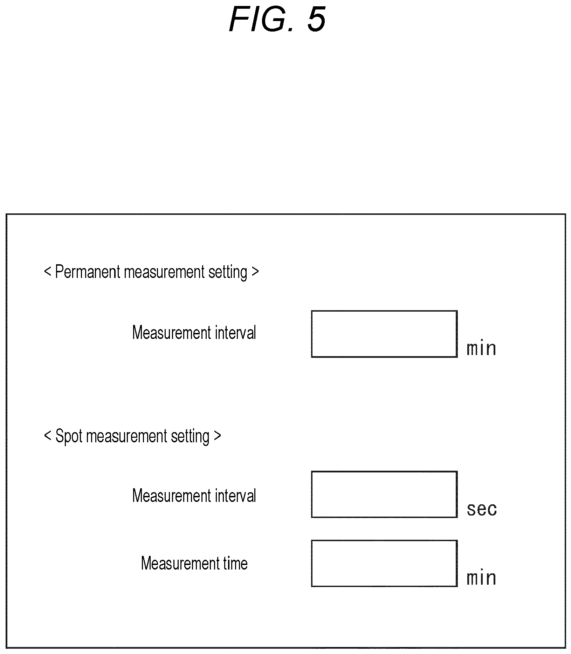

When a predetermined measurement condition is satisfied, each control apparatus 10 measures water quality by using the water quality sensor 30 connected to the control apparatus 10. The measurement condition is a condition for determining measurement timings. The measurement condition includes a measurement condition set beforehand and a condition which is satisfied upon receipt of an instruction from the outside (for example, the storage terminal 40). Setting of the measurement condition is performed, for example, by the storage terminal 40; more specifically, by a display image shown in FIG. 5. The measurement condition is set for each of the above-described measurement modes.

A measurement period is set as the measurement condition for the "permanent mode," and the measurement condition is satisfied every time the set measurement period elapses. Specifically, the measurement condition for the "permanent mode" is set as a result of entry of a numerical value into an input field for the "measurement interval" provided for "permanent measurement setting" of FIG. 5. The "measurement interval" is set, for example, in the unit of minute. An integer value (for example, 1 to 10) is entered in the input field for the "measurement interval." The entered value is set as the measurement period.

Also, a measurement start time is set as the measurement condition for the "spot mode," and the measurement condition is satisfied when the time elapsed after connection of the water quality sensor 30 reaches a measurement start time set beforehand. Also, a measurement time is set in addition to the measurement start time. The control apparatus 10 starts measurement when the time elapsed after connection of the water quality sensor 30 reaches the set measurement start time and ends the measurement when the time elapsed after start of the measurement reaches a measurement time set beforehand. Namely, the control apparatus 10 automatically starts the measurement by the water quality sensor 30 when the water quality sensor 30 is connected to one of the input ports 20. Specifically, the measurement condition for the "spot mode" is set as a result of entry of numerical values into the input fields for the "measurement interval" and the "measurement time" provided for "spot measurement setting" of FIG. 5. The "measurement interval" is set, for example, in the unit of second. For example, any of 15, 30, 45, and 60 is entered in the input field for the "measurement interval." The entered value is set as the measurement start time. The "measurement time" is set, for example, in the unit of minute. An integer value (for example, 5 to 30) is entered in the input field for the "measurement time." In the "spot mode," the control apparatus 10 does not perform measurement until the water quality sensor 30 once removed after completion of measurement is connected again.

The measurement conditions set at the storage terminal 40 are output to control apparatuses 10 and are stored in the control apparatuses 10. FIG. 1 shows an example in which the measurement mode is assumed to be set to the "spot mode." Only one water quality sensor 30 is prepared for the shipment tank area SA, and only one water quality sensor 30 is prepared for the intermediate tank area CA. However, as described above, the water quality sensors 30 can be attached to and detached from the input ports 20, and the input ports 20A and 20B have the same shape. Therefore, a user can measure the quality of the breeding water in each of the tanks 90 by using one water quality sensor 30 commonly among the tanks 90.

Each control apparatus 10 determines, for each input port 20, whether or not the above-described measurement condition is satisfied. In the case where the control apparatus 10 determines that the measurement condition is satisfied, the control apparatus 10 instructs the water quality sensor 30 connected to the input port 20, for which the control apparatus 10 determines that the measurement condition is satisfied, to measure water quality. The water quality sensor 30 having received the measurement instruction starts the measurement of water quality. Items to be measured are determined beforehand in accordance with the type of the water quality sensor 30. When the water quality sensor 30 ends the measurement of water quality, the water quality sensor 30 outputs a measurement value. Upon receipt of the measurement value from the water quality sensor 30, the control apparatus 10 stores and outputs to the outside the measurement value and the piece of identification information of the input port 20 through which the measurement value was received, in such a manner that the measurement value and the piece of identification information of the input port 20 are related to each other. Also, in the case where the control apparatus 10 has received from the water quality sensor 30 a piece of type information representing the type of the water quality sensor 30, the control apparatus 10 stores and outputs to the outside the measurement value, the piece of identification information of the input port 20, and the piece of type information in such a manner that the measurement value, the piece of identification information, and the piece of type information are related to one another.

In the case where the storage terminal 40 has received measurement values output from the control apparatuses 10, respectively, the storage terminal 40 stores pieces of water quality information based on the received measurement values while distinguishing them from one another on the basis of the pieces of identification information of the input ports 20 related to the measurement values. Specifically, on the basis of the above-described correspondence relationship data and the piece of identification information of the input port 20 related to each measurement value, the storage terminal 40 stores the piece of water quality information based on the measurement value in such a manner that the piece of water quality information is related to the tank 90 where that measurement value was measured. The "piece of water quality information based on the measurement value" may be the measurement value itself or a piece of information obtained by processing (for example, correcting) the measurement value. When the measurement value is processed, the measurement value may be processed by taking the type of the water quality sensor 30 into account.

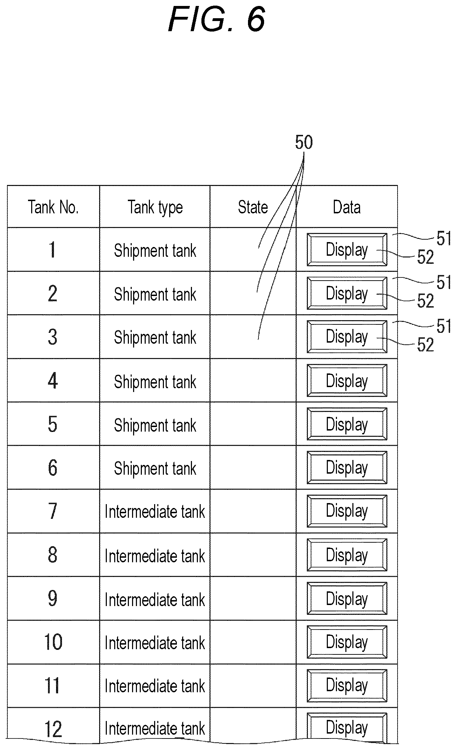

The storage terminal 40 can display on the display section 45 a display image illustrated in FIG. 6. On the display image illustrated in FIG. 6, "tank numbers" and a "tank type," a "state," and "data" corresponding to each of the "tank numbers" are displayed. Each of display fields 50 for "state" shows a state by, for example, its display color or its state of lighting (a lighted state or a flashing state). Examples of "state" include a state in which measurement is performed in the spot mode, a state after completion of measurement in the spot mode, a state in which measurement is waited in the permanent mode, a state in which measurement is performed in the permanent mode, a state in which communication with the control apparatus 10 is not established, a state in which the water quality sensor 30 is not connected, a state in which the measurement value is anomalous, and a state in which the control apparatus 10 is anomalous. Display buttons 52 are displayed in display fields 51 for "data."

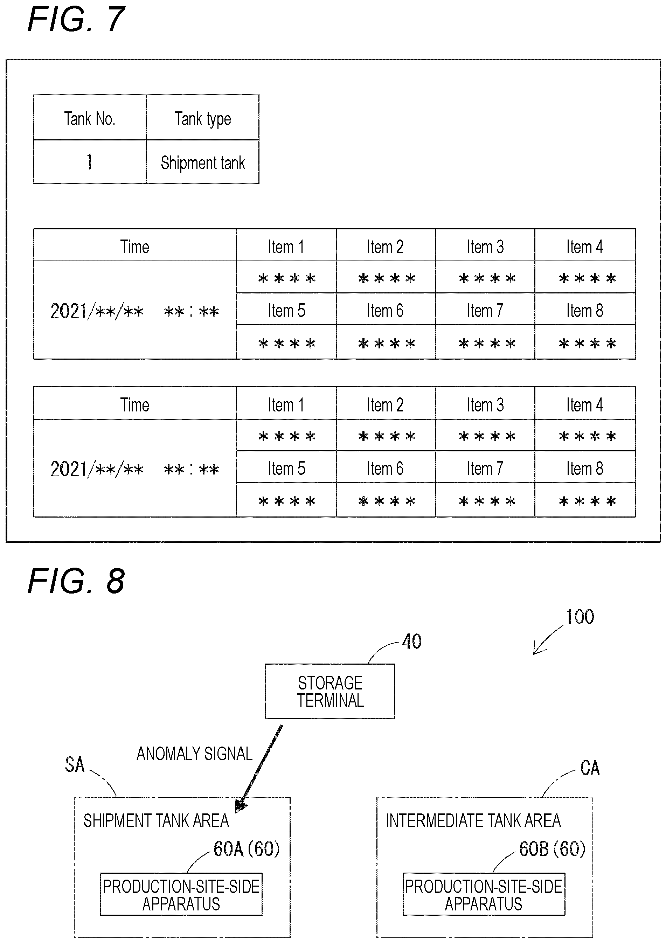

When a display button 52 is operated, as shown in FIG. 7, the storage terminal 40 displays, together with the "tank No." and the "tank type" of a tank 90 corresponding to the operated display button 52, "times" at which measurement was performed in that tank 90 and pieces of water quality information representing various items measured at each of the times.

The storage terminal 40 can perform different controls in accordance with the measurement mode. For example, in the case where the storage terminal 40 determines that the water quality sensor 30 is not connected in the permanent mode, the storage terminal 40 reports an anomaly. The storage terminal 40 reports the anomaly by a pop-up display on the display section 45. When the storage terminal 40 reports the anomaly, the storage terminal 40 also displays a piece of information representing the input port 20 to which the water quality sensor 30 is not connected.

The storage terminal 40 determines whether or not each piece of water quality information is anomalous. In the case where the storage terminal 40 determines that the piece of water quality information is anomalous, the storage terminal 40 causes the anomaly to be reported at a production site where the measurement value, from which that piece of water quality information was obtained, was generated. As shown in FIG. 8, the water quality management system 100 has a production-site-side apparatus 60 at each production site. Specifically, a production-site-side apparatus 60A is disposed in the shipment tank area SA, and a production-site-side apparatus 60B is disposed in the intermediate tank area CA.

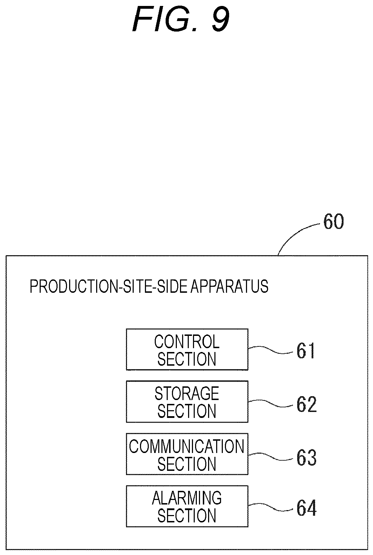

As shown in FIG. 9, each production-site-side apparatus 60 includes a control section 61, a storage section 62, a communication section 63, and an alarming section 64. The control section 61 is, for example, an information processing apparatus including a CPU and can perform various types of computations, controls, and information processing. The storage section 62 is, for example, a memory such as ROM, RAM, or the like and stores various pieces of information. The communication section 63 is an apparatus which communicates with an external apparatus in accordance with a known scheme. Although the communication scheme employed in the present embodiment is radio communication, the communication scheme may be wire communication. The alarming section 64 has a function of reporting an anomaly when it occurs and is, for example, a well known display apparatus such as a display. The alarming section 64 reports the anomaly by, for example, pop-up display.

In the case where a certain piece of water quality information represents an anomalous value, the storage terminal 40 outputs an anomaly signal to the production-site-side apparatus 60 at the production site where the measurement value, from which that piece of water quality information was obtained, was generated. Upon receipt of the anomaly signal, the production-site-side apparatus 60 reports the anomaly by the alarming section 64.

### 1-4. Examples of effects of the present configuration

Each control apparatus 10 is configured in such a manner that the control apparatus 10 can be connected to the water quality sensor 30 which is disposed in each of the plurality of tank 90. The control apparatus 10 includes a plurality of input ports 20 to which the water quality sensor 30 is detachably connected. The control apparatus 10 receives a measurement value from the water quality sensor 30 connected to one of the input ports 20, and stores and outputs to the outside the measurement value and a piece of identification information of the input port 20 through which the measurement value is received, in such a manner that the measurement value and the piece of identification information of the input port 20 are related to each other. This control apparatus 10 can store and output to the outside the measurement value from the water quality sensor 30 and the piece of identification information of the input port 20 through which the measurement value is received, in such a manner that the measurement value and the piece of identification information of the input port 20 are related to each other. Therefore, a plurality of pieces of water quality information based on the measurement values obtained at the plurality of tanks 90 can be managed individually by a single control apparatus 10 on a tank-by-tank basis. Accordingly, by virtue of this configuration, it is possible to manage pieces of information relating to water quality and obtained at the plurality of tanks 90, while suppressing an increase in cost and reducing the labor of an operator.

Furthermore, the plurality of input ports 20 of each control apparatus 10 are identical in shape. This control apparatus 10 enables connection of the same water quality sensor 30 to the plurality of input ports 20 as a common water quality sensor. Therefore, the number of water quality sensors 30 needed for measurement can be reduced.

Furthermore, the control apparatus 10 can receive, from the water quality sensor 30 connected to the input port 20, a piece of type information representing the type of the water quality sensor 30. By virtue of this configuration, it is possible to manage pieces of water quality information in consideration of the type of the water quality sensor 30.

Furthermore, the water quality management system 100 includes the control apparatus 10 and the storage terminal 40. The storage terminal 40 stores pieces of water quality information based on the measurement values output from the control apparatus 10 in such a manner that the pieces of water quality information are distinguished from one another on the basis of pieces of identification information of the input ports 20 related to the measurement values. In this water quality management system 100, since the pieces of water quality information based on the measurement values output from the control apparatus 10 can be stored in such a manner that the pieces of water quality information are automatically distinguished from one another on the basis of pieces of identification information of the input ports 20, the pieces of water quality information obtained from the tanks 90 individually can be managed easily.

Furthermore, in the water quality management system 100, the storage terminal 40 can set the measurement mode to the permanent mode in which the water quality sensor 30 is permanently connected to one of the input ports 20 or the spot mode in which the water quality sensor 30 is connected to one of the input ports 20 when necessary. By virtue of this configuration, it is possible to perform more appropriate control depending on whether the measurement mode is the permanent mode or the spot mode.

Furthermore, in the water quality management system 100, the storage terminal 40 is configured such that, in the case where the storage terminal 40 determines that the water quality sensor 30 is not connected in the permanent mode, the storage terminal 40 reports an anomaly. By virtue of this configuration, it is possible to report an anomaly in the case where the water quality sensor 30 is determined not to be connected despite the measurement mode being the permanent mode.

Furthermore, in the water quality management system 100, the storage terminal 40 enables setting for establishing correspondence relationships between the pieces of identification information of the tanks 90 and the pieces of identification information of the control apparatus 10 and the input ports 20.

By virtue of this configuration, the storage terminal 40 can manage the pieces of water quality information based on the measurement values received from the control apparatus 10 in such a manner that the pieces of water quality information are related to the tanks 90.

Furthermore, in the water quality management system 100, a plurality of control apparatuses 10 are provided. The storage terminal 40 includes the display section 45 and causes the display section 45 to display the pieces of water quality information based on the measurement values received from the plurality of control apparatuses 10. By virtue of this configuration, the pieces of water quality information based on the measurement values output from the plurality of control apparatuses 10 can be displayed by the display section 45 of the storage terminal 40. Therefore, a managing person can check, by using the storage terminal 40, the pieces of water quality information obtained at the respective tanks 90.

Furthermore, in the water quality management system 100, the storage terminal 40 can set measurement conditions for the control apparatus 10 under which the water quality sensor 30 connected to the control apparatus 10 performs measurement. By virtue of this configuration, the conditions under which the water quality sensor 30 performs measurement can be set not on the control apparatus 10 side but on the storage terminal 40. Therefore, the managing person can set the measurement conditions at the respective tanks 90 by using the storage terminal 40, and, thus, it is possible to eliminate time and labor; such as for instructing a person at the site to set the measurement conditions.

Furthermore, the water quality management system 100 includes the production-site-side apparatuses 60 provided at the respective production sites where the control apparatuses 10 are disposed. In the case where the storage terminal 40 determines that a piece of water quality information based on the measurement value from the water quality sensor 30 is anomalous, the storage terminal 40 outputs an anomaly signal to the production-site-side apparatus 60 provided at the production site where the measurement value, from which that piece of water quality information was obtained, was generated. Upon receipt of the anomaly signal, the production-site-side apparatus 60 reports the anomaly. By virtue of this configuration, in the case where the measurement value form the water quality sensor 30 is an anomalous value, the anomaly can be reported at the production site where the control apparatus 10 is disposed.

### <Other embodiments>

The present invention is not limited to the embodiment described by the above description and the drawings, and, for example, the following embodiments fall within the technical scope of the present invention. Also, various features of the above-described embodiment and the following embodiments may be combined freely so long as no conflict occurs.

In the above-described embodiment, the storage terminal is a stationary device (e.g., a computer). However, the storage terminal may be a portable device such as a smartphone, a tablet, or the like.

In the above-described embodiment, the water quality sensor is a multi-parameter water quality meter. However, the water quality sensor may be a sensor which measures only one item.

The above-described embodiment is configured in such a manner that, in the case where the storage terminal determines that a piece of water quality information based on a measurement value from a water quality sensor is anomalous, the storage terminal outputs an anomaly signal to the production-site-side apparatus, and the production-site-side apparatus reports the anomaly. However, the embodiment may be configured in such a manner that the storage terminal outputs an anomaly signal to a control apparatus, and the control apparatus reports the anomaly. Alternatively, the embodiment may be configured to report the anomaly by an image displayed on the display section of the storage terminal, a sound output from the sound output section thereof, or the like.

The above-described embodiment is configured in such a manner that one input port is related to one tank. However, the embodiment may be configured in such a manner that a plurality of input ports are related to one tank. Examples of the configuration in which a plurality of input ports are related to one tank include a configuration in which water quality sensors of different types are connected to the plurality of input ports, respectively, and a configuration in which water quality sensors which measure different items are connected to the plurality of input ports, respectively.

Notably, the embodiments disclosed this time should be considered to be illustrative and not to be restrictive in all aspects. The scope of the present invention is not limited to the embodiments disclosed this time, and it is intended that the present invention encompasses all modifications within the range shown by the claims and the range of equivalents of the claims.

### REFERENCE SIGNS LIST

10: control apparatus
10A: control apparatus
10B: control apparatus
10C: control apparatus
10D: control apparatus
10E: control apparatus
10F: control apparatus
20: input port
20A: input port
20B: input port
30: water quality sensor
30A: water quality sensor
30B: water quality sensor
40: storage terminal
45: display section
60: production-site-side apparatus
60A: production-site-side apparatus
60B: production-site-side apparatus
90: tank
90A: tank
90B: tank
91A: tank
91B: tank
92A: tank
92B: tank
93A: tank
93B: tank
94A: tank
94B: tank
95A: tank
95B: tank
100: water quality management system
101: water quality sensor unit
110: water quality management unit

## Claims

1. A control apparatus which can be connected to a water quality sensor, comprising:
a plurality of input ports to which the water quality sensor that can be disposed in a plurality of tanks whose number is equal to or less than the number of the input ports is detachably connected and each of which is related to any one of the plurality of tanks,
wherein the control apparatus receives a measurement value from the water quality sensor connected to one of the input ports, and stores or outputs to an outside the measurement value and a piece of identification information of the input port through which the measurement value is received, in such a manner that the measurement value and the piece of identification information of the input port are related to each other.

2. A control apparatus according to claim 1, wherein the plurality of input ports are identical in shape.

3. A control apparatus according to claim 1 or 2, wherein the control apparatus can receive a piece of type information from the water quality sensor connected to the input port, the piece of type information representing a type of the water quality sensor.

4. A water quality management system comprising:
a control apparatus according to any one of claims 1 to 3; and
a storage terminal which stores pieces of water quality information based on the measurement values output from the control apparatus, in such a manner that the pieces of water quality information are distinguished from each other on the basis of the pieces of identification information of the input ports through which the measurement values are received.

5. A water quality management system according to claim 4, wherein the storage terminal can set a measurement mode to a permanent mode in which the water quality sensor is permanently connected to one of the input ports, or a spot mode in which the water quality sensor is connected to one of the input ports when necessary.

6. A water quality management system according to claim 5, wherein, in the case where the storage terminal determines that the water quality sensor is not connected in the permanent mode, the storage terminal reports an anomaly.

7. A water quality management system according to any one of claims 4 to 6, wherein the storage terminal enables setting for establishing correspondence relationships between pieces of identification information of the tanks and pieces of identification information of the control apparatus and the input ports.

8. A water quality management system according to any one of claims 4 to 7, comprising a plurality of the control apparatuses, wherein the storage terminal has a display section and causes the display section to display pieces of water quality information based on the measurement values received from the control apparatuses, respectively.

9. A water quality management system according to any one of claims 4 to 8, wherein the storage terminal can set measurement conditions for the control apparatus, under which the water quality sensor connected to the control apparatus performs measurement.

10. A water quality management system according to any one of claims 4 to 9, wherein, in the case where the storage terminal determines that the piece of water quality information based on the measurement value from the water quality sensor is anomalous, the storage terminal outputs an anomaly signal.

11. A water quality management unit comprising:
the water quality sensor; and
a water quality management system according to any one of claims 4 to 10.

12. A water quality sensor unit comprising:
the water quality sensor; and
a control apparatus according to any one of claims 1 to 3.
